# EUROPÄISCHE PATENTSCHRIFT

(11) **EP 1 593 373 B1**
(45) Veröffentlichungstag und Bekanntmachung des Hinweises auf die Patenterteilung: **14.04.2010**
(21) Anmeldenummer: 05014387.4
(22) Anmeldetag: 06.06.2002
(51) Int. Cl.: A61K 8/34, A61K 8/41, A61Q 5/02

(54) **Neuartig konfektionierte quaternäre Ammoniumverbindungen**
Compositions comprising quaternary ammonium compounds
Compositions comprenant des ammoniums quaternaires

(30) Priorität: 19.06.2001 DE 10129225
(43) Veröffentlichungstag der Anmeldung: 09.11.2005
(62) Teilanmeldung aus: 02012371.7
(73) Patentinhaber: Clariant Produkte (Deutschland) GmbH, 65929 Frankfurt am Main (DE)
(72) Erfinder: Milbradt, Robert, Dr., 65191 Wiesbaden (DE); Klein, Sonja, 65439 Flörsheim (DE); Scherl, Franz Xaver, Dr., 84508 Burgkirchen (DE); Gatter, Erich, Dr., 84556 Kastl (DE); Oberhauser, Adelgunde, 84524 Neuötting (DE)

(56) Entgegenhaltungen:
- EP-A- 1 262 171
- WO-A-00/28950
- US-A- 4 069 347

## Beschreibung

Die Erfindung betrifft Zusammensetzungen, enthaltend quaternäre Ammoniumverbindungen, die einen niedrigen Stockpunkt, gute Löslichkeit bzw. Dispergierbarkeit in wässrigen Medien und einen niedrigen Flammpunkt besitzen und somit hervorragend zur Konfektionierung von quaternären Ammoniumverbindungen geeignet sind.

Kosmetische Mittel, wie z.B. Haarbehandlungsmittel, enthalten häufig in Wasser schwerlösliche quaternäre Ammoniumverbindungen, die eine langkettige Alkyl- oder Alkenylgruppe aufweisen. Solche Mittel sind üblicherweise als wässrige Dispersionen, Emulsionen, Mikroemulsionen, Gele oder auch in Aerosolform konfektioniert und werden z.B. als Shampoos, Haarkuren, Haarspülungen etc. eingesetzt.

Für den Hersteller solcher Mittel ist es von großem Vorteil, die quaternären Ammoniumverbindungen als Compounds oder Formulierungen in Form von Flakes, Pellets oder Pasten bereitzustellen, die bei hohem kationischen Wirkstoffanteil einen niedrigen Stockpunkt sowie gute Löslichkeit bzw. Dispergierbarkeit in wässrigen Medien besitzen.
Gemäß dem Stand der Technik können die obigen Anforderungen durch die Zugabe von kurzkettigen Alkoholen, insbesondere von Isopropanol in Mengen von 15 bis 20 Gew.-%, erreicht werden. Aufgrund ihrer niedrigen Siede- und Flammpunkte sind solche kurzkettigen Alkohole jedoch problematisch.
Wie in WO 00/28950 beschrieben, können die kurzkettigen Alkohole durch lineare Fettalkohole (z.B. Cetyl-, Lauryl-, Behenyl- oder Stearylalkohol) ersetzt werden. Um den Stockpunkt bzw. Schmelzpunkt der Mischungen auf Temperaturen unterhalb 100°C herabzusetzen werden zusätzlich Glykole, wie z.B. Propylenglykol oder 1,3-Butandiol, zugesetzt.

In der WO 00/28950 wird weiterhin hervorgehoben, dass es sich bei den Fettalkoholen vorteilhafterweise um homogene Fettalkohole handelt, die weniger als ca. 10 Gew.-% eines anderen Fettalkohols enthalten.
In der WO 00/28950 wird weiterhin ausgeführt, dass der Ersatz der kurzkettigen Alkohole durch Glykole ohne gleichzeitige Zugabe von Fettalkoholen zu nicht pelletierbaren Formulierungen führt.

Die ältere Anmeldung EP 1 262 171 beschreibt Zusammensetzungen, die mindestens eine quaternäre Ammoniumverbindung, mindestens einen verzweigten Alkohol sowie mindestens einen mehrwertigen Alkohol mit 2 bis 6 C-Atomen enthalten. US 4 069 347 beschreibt Mischungen aus 25 bis 65 Gew.-% Lanolinamidoalkylammonium-Verbindungen und 35 bis 75 Gew.-% eines aliphatischen, gesättigten und verzweigten Diols. Diese Mischungen werden in kosmetischen Zubereitungen (Shampoos, Haarspülungen) eingesetzt.

Überraschenderweise wurde nun gefunden, dass Zusammensetzungen, wie im Folgenden definiert, niedrige Stock- und Schmelzpunkte, gute Löslichkeit bzw. Dispergierbarkeit in wässrigen Medien und einen niedrigen Flammpunkt besitzen.

Überraschenderweise sind die Zusammensetzungen bei Raumtemperatur ausreichend hart und spröde, um pelletiert oder geschuppt zu werden. Die neuartigen Zusammensetzungen eignen sich damit hervorragend zur Konfektionierung von quaternären Ammoniumverbindungen.

Gegenstand der Erfindung sind Zusammensetzungen, enthaltend
a) mindestens eine quaternäre Ammoniumverbindung gemäß Formel (1) 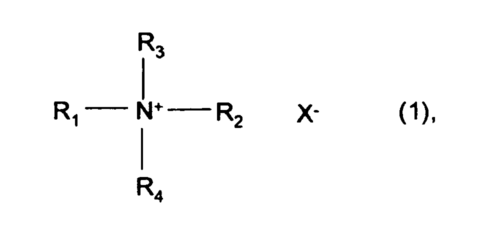 wobei
   R₁ für eine unverzweigte oder verzweigte Alkyl- oder Alkenylgruppe mit 12 bis 36 Kohlenstoffatomen, eine Gruppe R₅CONH(CH₂)ₙ- oder eine Gruppe R₅COO(CH₂)ₙ-steht, wobei R₅ für eine Alkyl- oder Alkenylgruppe mit 12 bis 36 Kohlenstoffatomen und n für eine Zahl von 1 bis 8 stehen,
   und
   R₂, R₃ und R₄ unabhängig voneinander gleich oder verschieden sein können und für eine -CH₃, CH₃CH₂-,CH₃CH₂CH₂-, CH₃CH₂CH₂CH₂-, -CH₂CH₂OH oder -CH₂CH(OH)CH₂OH-Gruppe stehen,
   X⁻ für ein Anion steht,
   und mindestens eine Ammoniumverbindung a) ausgewählt ist aus Alkyltrimethylammoniumverbindungen bei denen der Alkylrest einen Behenyl-, Erucyl-, Cetyl- oder Stearylrest darstellt,
   und
b) mindestens einen mehrwertigen Alkohol mit 5 bis 12 Kohlenstoffatomen, dadurch gekennzeichnet, dass der Gehalt an quaternären Ammoniumverbindungen a), bezogen auf die fertigen Zusammensetzungen, 60 bis 90 Gew.-% beträgt und die Zusammensetzungen in Form von Pellets oder Flakes vorliegen, mit der Maßgabe, dass die Zusammensetzungen frei sind von verzweigten Alkoholen b1) mit 8 bis 26 Kohlenstoffatomen oder Mischungen aus mindestens einem verzweigten Alkohol b1) und mindestens einem unverzweigten Alkohol b2) mit 8 bis 36 Kohlenstoffatomen, wenn sie mindestens einen mehrwertigen Alkohol mit 2 bis 6 Kohlenstoffatomen enthalten.

In einer bevorzugten Ausführungsform sind die Zusammensetzungen frei von Fettalkoholen und linearen oder verzweigten Monoalkoholen mit 8 bis 36 Kohlenstoffatomen.

Der Anteil an quaternären Ammoniumverbindungen a), bezogen auf die fertigen Zusammensetzungen, beträgt 60 bis 90 Gew.-%, bevorzugt 60 bis 85 Gew.-%. Überraschenderweise zeigte sich, dass die Zusammensetzungen vorteilhafterweise hohe Gewichtsanteile an quaternären Ammoniumverbindungen a) bei gleichzeitig niedrigen Schmelz- bzw. Stockpunkten enthalten können.

Als quaternäre Ammoniumverbindungen a) bevorzugt sind
(C₁₂-C₃₆)-Alkyltrimethylammoniumverbindungen,
besonders bevorzugt (C₁₄-C₃₀)-Alkyltrimethylammoniumverbindungen, insbesondere bevorzugt (C₁₆-C₂₄)-Alkyltrimethylammoniumverbindungen.

Beim Anion X⁻ in Formel (1) kann es sich um ein beliebiges ladungsausgleichendes Anion handeln; bevorzugt sind Chlorid, lodid, Bromid, Methosulfat, Hydrogensulfat, Lactat und/oder Citrat, besonders bevorzugt Chlorid und Methosulfat.

Ganz besonders geeignet als quaternäre Ammoniumverbindung a) ist Behenyltrimethylammoniumchlorid.

Der Anteil an mehrwertigen Alkoholen b) beträgt, bezogen auf die fertigen Zusammensetzungen, bevorzugt 10 bis 70 Gew.-%, besonders bevorzugt 15 bis 60 Gew.-%, insbesondere bevorzugt 15 bis 55 Gew.-%.

Unter mehrwertigen Alkoholen b) sind solche zu verstehen, die mindestens zwei OH-Gruppen im Molekül tragen. Die mehrwertigen Alkohole b) können unverzweigt oder verzweigt und gesättigt oder ungesättigt sein. Weiterhin können die mehrwertigen Alkohole b) aus über Etherbrücken verknüpften niedermolekularen mehrwertigen Alkoholen aufgebaut sein.

Als mehrwertige Alkohole b) bevorzugt geeignet sind Pentandiol, Hexandiol, Hexylenglykol, Trimethylpentandiol, Heptandiol, Octandiol, Nonandiol, Decandiol, Undecandiol, Dodecandiol, Diglycerin, Triglycerin, Dipropylenglykol, Tripropylenglykol, Sorbitol, Xylitol, Mannitol und/oder Mischungen derselben.

Besonders bevorzugt als mehrwertige Alkohole b) sind 1,5-Pentandiol, 1,2-Pentandiol, 1,6-Hexandiol, 1,2-Hexandiol, 1,7-Heptandiol, 1,2-Heptandiol, 1,8-Octandiol, 1,2-Octandiol, 1,9-Nonandiol, 1,2-Nonandiol, 1,10-Decandiol, 1,2-Decandiol, 1,11-Undecandiol, 1,2-Undecandiol, 1,12-Dodecandiol, 1,2-Dodecandiol, 2-Methyl-2,4-pentandiol, 2,2,4-Trimethyl-1,3-pentandiol, Diglycerin, Triglycerin, Dipropylenglykol, Tripropylenglykol, Sorbitol, Xylitol, Mannitol und/oder Mischungen derselben.

Insbesondere bevorzugt als mehrwertige Alkohole b) sind 1,6-Hexandiol, Dipropylenglykol, 2-Methyl-2,4-pentandiol, 2,2,4-Trimethyl-1,3-pentandiol und Mischungen derselben.

Besonders vorteilhafte anwendungstechnische Eigenschaften zeigen Zusammensetzungen, die mindestens einen mehrwertigen Alkohol b) mit 6 bis 8 Kohlenstoffatomen enthalten, der gegebenenfalls zusammen mit anderen mehrwertigen Alkoholen b) eingesetzt werden kann.

Optional können die erfindungsgemäßen Zusammensetzungen zur Verbesserung der anwendungstechnischen Eigenschaften unverzweigte oder verzweigte Monoalkohole mit 1 bis 4 Kohlenstoffatomen enthalten.
Bevorzugt als Monoalkohole sind Ethanol, Propanol, Isopropanol, Butanol, Isobutanol und t-Butanol, besonders bevorzugt Isopropanol.
Bevorzugt enthalten die Zusammensetzungen, bezogen auf die fertigen Zusammensetzungen, weniger als 5 Gew.-%, besonders bevorzugt weniger als 3 Gew.-%, an derartigen Monoalkoholen.
In einer bevorzugten Ausführungsform sind die Zusammensetzungen frei von unverzweigten oder verzweigten Monoalkoholen mit 1 bis 4 Kohlenstoffatomen.

Die erfindungsgemäßen Zusammensetzungen besitzen bevorzugt Stockpunkte unterhalb 100°C, besonders bevorzugt unterhalb 95°C, insbesondere bevorzugt unterhalb 90°C, ganz besonders bevorzugt unterhalb 85°C.

Die Flammpunkte der erfindungsgemäßen Zusammensetzungen liegen bevorzugt oberhalb 80°C, besonders bevorzugt oberhalb 100°C.

Bei den erfindungsgemäßen Zusammensetzungen handelt es sich um Pellets oder Flakes.

In einer bevorzugten Ausführungsform werden die erfindungsgemäßen Zusammensetzungen dadurch hergestellt, dass man mindestens eine quaternäre Ammoniumverbindung a), gegebenenfalls enthaltend einen verzweigten oder unverzweigten Monoalkohol mit 1 bis 4 Kohlenstoffatomen, und mindestens einen mehrwertigen Alkohol b) mit 5 bis 12 Kohlenstoffatomen und gegebenenfalls mindestens einen unverzweigten oder verzweigten Monoalkohol mit 1 bis 4 Kohlenstoffatomen mischt und diese Mischung in Form von Pellets oder Flakes bringt.

In einer bevorzugten Ausführungsform werden die Komponenten i) bis iii) vermischt und anschließend, gegebenenfalls unter Rühren, erwärmt. Dabei wird die Temperatur so gewählt, dass eine Schmelze vorliegt. Bevorzugt sind Temperaturen von 70 bis 120 °C, besonders bevorzugt 80 bis 110°C. In einer anderen bevorzugten Ausführungsform wird die Komponente i) als Schmelze vorgelegt.
Die quaternären Ammoniumverbindungen a) der Komponente i) können in bekannter Weise durch Alkylierung eines tertiären Amins in Gegenwart mindestens eines unverzweigten oder verzweigten Monoalkohols mit 1 bis 4 Kohlenstoffatomen, bevorzugt Ethanol, Propanol, Isopropanol, Butanol, Isobutanol und t-Butanol, besonders bevorzugt Isopropanol, hergestellt werden. Die quaternären Ammoniumverbindungen werden bevorzugt als Pellets oder besonders bevorzugt als Pulver eingesetzt. In einer bevorzugten Ausführungsform enthalten die quaternären Ammoniumverbindungen weniger als 5 Gew.-%, bevorzugt weniger als 3 Gew.-%, an Monoalkoholen. In einer weiteren bevorzugten Ausführungsform enthalten die quaternären Ammoniumverbindungen 10 bis 25 Gew.-% an Monoalkoholen.
Die erfindungsgemäßen Zusammensetzungen besitzen bevorzugt einen Gesamtgehalt an unverzweigten oder verzweigten Monoalkoholen mit 1 bis 4 Kohlenstoffatomen von weniger als 5 Gew.-%, besonders bevorzugt weniger als 3 Gew.-%.
In einer ebenfalls bevorzugten Ausführungsform sind die erfindungsgemäßen Zusammensetzungen frei von unverzweigten oder verzweigten Monoalkoholen mit 1 bis 4 Kohlenstoffatomen.
Zur Einstellung des gewünschten Gehalts an Monoalkoholen in den erfindungsgemäßen Zusammensetzungen werden die Komponenten i) und/oder iii) entsprechend gewählt bzw. bemessen und/oder man entfernt die Monoalkohole teilweise oder ganz vorab aus der Komponente i).
In einer weiteren Ausführungsform werden die Monoalkohole nachträglich aus den erfindungsgemäßen Zusammensetzungen bis auf den gewünschten Restgehalt entfernt. Bevorzugt werden die Monoalkohole bei 700 bis 10 mbar, bevorzugt 400 bis 70 mbar, und 60 bis 90°C abgezogen.
Ebenso können die Monoalkohole bei Normaldruck in geeigneten Verdampfungseinrichtungen (z.B. Dünnschicht-Verdampfer) bei Temperaturen bis 120 °C abdestilliert werden.

Überraschenderweise wurde gefunden, dass die erfindungsgemäßen Zusammensetzungen auch "in situ" durch Alkylierung von
i) mindestens einem tertiären Amin NR₁R₂R₃,
   wobei R₁ für eine unverzweigte oder verzweigte Alkyl- oder Alkenylgruppe mit 12 bis 36 Kohlenstoffatomen, eine Gruppe R₅CONH(CH₂)ₙ- oder eine Gruppe R₅COO(CH₂)ₙ-steht, wobei R₅ für eine Alkyl- oder Alkenylgruppe mit 12 bis 36 Kohlenstoffatomen und n für eine Zahl von 1 bis 8 stehen, und R₂ und R₃ unabhängig voneinander gleich oder verschieden sein können und für CH₃-, CH₃CH₂-, CH₃CH₂CH₂-, CH₃CH₂CH₂CH₂- oder -CH₂CH₂(OH) stehen, durch
ii) mindestens ein Alkylierungsmittel, ausgewählt aus
   a) R₄X, wobei R₄ für -CH₃, CH₃CH₂-, CH₃CH₂CH₂-, CH₃CH₂CH₂CH₂- oder -CH₂CH(OH)CH₂(OH) steht und X für Cl, I, Br, OSO₃H oder Methosulfat steht, und/oder
   b) Ethylenoxid und einer Säure HX, wobei X für Cl, I, Br, OSO₃H, Citrat oder Lactat steht,
   in Gegenwart von
iii) mindestens einem mehrwertigen Alkohol b) mit 5 bis 12 Kohlenstoffatomen und
iv) gegebenenfalls mindestens einem unverzweigten oder verzweigten Monoalkohol mit 1 bis 4 Kohlenstoffatomen
hergestellt werden können. Abschließend werden diese Mischungen in die Form von Flakes oder Pellets gebracht.

Bevorzugt werden bei der Umsetzung die Einsatzmengen an mehrwertigen Alkoholen b) und gegebenenfalls unverzweigten oder verzweigten Monoalkoholen mit 1 bis 4 Kohlenstoffatomen entsprechend den schon vorab beschriebenen bevorzugten Zusammensetzungen der erfindungsgemäßen Zusammensetzungen gewählt. Die Gehalte können auch durch nachträgliche Zugabe oder Entfernen der Komponenten eingestellt werden.

In einer bevorzugten Ausführungsform werden bei der Umsetzung keine unverzweigten oder verzweigten Monoalkohole mit 1 bis 4 Kohlenstoffatomen eingesetzt.

Die nach den oben beschriebenen Verfahren hergestellten erfindungsgemäßen Zusammensetzungen werden als homogene oder inhomogene Schmelze durch Abkühlen in Pellets oder Flakes, überführt.

Die erfindungsgemäßen Zusammensetzungen eignen sich allgemein zur Herstellung von Mitteln, enthaltend quaternäre Ammoniumverbindungen.
Besonders geeignet sind die Zusammensetzungen zur Herstellung von kosmetischen, dermatologischen und pharmazeutischen Mitteln.
Insbesondere eignen sich die Zusammensetzungen zur Herstellung von Haarbehandlungsmitteln.

Gegenstand der Erfindung ist demnach auch die Verwendung der erfindungsgemäßen Zusammensetzungen zur Herstellung von Mitteln, bevorzugt kosmetischen, dermatologischen und pharmazeutischen Mitteln, insbesondere Haarbehandlungsmitteln, enthaltend quaternäre Ammoniumverbindungen.

Beispiele für bevorzugte Mittel sind Shampoos, rinse-off-Haarconditionierer, Cremespülungen, Klarspülungen, Haarkuren, Haarfärbe- und Haartönungsmittel, Dauerwellmittel, Haargele, Haarconditionierer in Aerosol-, Spray- und Fluidform, 2-in-1 Duschbäder, Cremeduschbäder, Hautpflegemittel, Tagescremes, Nachtcremes, Pflegecremes, Nährcremes, Bodylotions und Salben.

Die kosmetischen, dermatologischen und pharmazeutischen Mittel enthalten die erfindungsgemäßen Zusammensetzungen, bezogen auf die fertigen Mittel, bevorzugt in Mengen von 0,1 bis 15 Gew.-%, besonders bevorzugt 1 bis 10 Gew.-%, insbesondere bevorzugt 1 bis 7 Gew.-%.

Die kosmetischen, dermatologischen und pharmazeutischen Mittel können als weitere Hilfs- und Zusatzstoffe alle üblichen Tenside, Ölkörper, Emulgatoren und Co-Emulgatoren, kationischen Polymere, Filmbildner, Überfettungsmittel, feuchtigkeitsspendende Mittel, Stabilisatoren, biogene Wirkstoffe, Konservierungsmittel, Perlglanzmittel, Farb- und Duftstoffe, Lösungsmittel, Glycerin, Hydrotrope, Trübungsmittel, Verdickungsmittel, Dispergiermittel, Eiweißderivate, wie z.B. Gelatine, Collagenhydrolysate, Polypeptide auf natürlicher und synthetischer Basis, Eigelb, Lecithin, Lanolin und Lanolinderivate, Silicone, deodorierende Mittel, Stoffe mit keratolytischer und keratoplastischer Wirkung, Enzyme, Trägersubstanzen, Antioxidantien, UV-Lichtschutzfilter, Pigmente und Metalloxide, sowie antimikrobiell wirkende Agentien enthalten.

Als Tenside können anionische, kationische, nichtionische, amphotere und/oder zwitterionische Tenside verwendet werden.
Bevorzugte nichtionische Tenside enthalten als hydrophile Gruppe eine Polyolgruppe, eine Polyolalkenylethergruppe oder eine Kombination aus Polyol- und Polyglykolethergruppen. Bevorzugt sind Anlagerungsprodukte aus von 2 bis 30 Mol Ethylenoxid, 2 bis 30 Mol Ethylenoxid zusammen mit bis 5 Mol Propylenoxid oder von bis 5 Mol Propylenoxid an lineare Fettalkohole mit 8 bis 22 C-Atomen und Alkylphenolen mit 8 bis 15 C-Atomen in der Alkylgruppe, (C₁₂-C₁₉)-Fettsäuremono- und diester von Anlagerungsprodukten von 1 bis 30 Mol Ethylenoxid an Glycerin, Glycerinmono- und -diester und Sorbitanmono- und -diester von gesättigten und ungesättigten (C₈-C₁₈)-Fettsäuren und deren Ethylenoxidanlagerungsprodukte, (C₈-C₁₈)-Alkylmono- und -oligoglykoside und deren ethoxylierte Analoga, Anlagerungsprodukte von 10 bis 60 Mol Ethylenoxid an Ricinusöl und gehärtetes Ricinusöl, ethoxylierte und nicht ethoxylierte Mono-, Di- und Trialkylmonophosphorsäureester, insbesondere Mono-, Di- und Tri-(Lauryltetraglycolether)-o-Phosphor-säureester und Mono-, Di- und Tri-(Cetyltetraglycolether)-o-Phosphorsäureester.

Bevorzugte amphotere Tenside tragen eine (C₈-C₁₈)-Alkyl- oder -Acylgruppe und mindestens eine freie Aminogruppe und mindestens eine -COOH- oder -SO₃H-Gruppe. Bevorzugt sind N-Acylglycine, N-Alkylpropionsäure, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils 8 bis 18 C-Atomen in der Alkylgruppe.
Besonders bevorzugte sind N-Kokosalkylaminopropionat, Kokosacylaminoethylaminopropionat und (C₁₂-C₁₈)-Alkylsarcosine.

Besonders geeignete zwitterionische Tenside sind Betaine, wie beispielsweise die N-Alkyl-N,N-dimethylammoniumglycinate, z.B. Kokosalkyldimethylammoniumglycinate, N-Acyl-aminopropyl-N-N-dimethylammoniumglycinate, z.B. Kokosacylaminopropyl-dimethylammoniumglycinat, 2-Alkyl-3-carboxymethyl-3-hydroxyethylimidazoline mit jeweils 8 bis 18 C-Atomen in der Alkyl- oder Acylgruppe und das Kokosacylaminoethylhydroxyethylcarboxymethylglycinat.

Bevorzugt enthalten die Mittel Tensid-Mischungen, besonders bevorzugt sind Mischungen aus nichtionischen und zwitterionischen oder amphoteren Tensiden in einem Gewichtsverhältnis von 5 zu 1 bis 1 zu 5 oder Mischungen aus nichtionogenen Tensiden und beliebigen Mischungen aus zwitterionischen und amphoteren Tensiden in einem Gewichtsverhältnis von 5 zu 1 bis 1 zu 5.

Als Ölkörper eignen sich alle bekannten Öle, Fette und Wachse mineralischer, tierischer, pflanzlicher und synthetischer Herkunft. Bevorzugt sind als Öl- und Fettkomponenten Diallylether mit insgesamt 12 bis 24 C-Atomen, Fettsäureester mit insgesamt 12 bis 26 C-Atomen, flüssige Kohlenwasserstoffe mit 10 bis 32 C-Atomen und Gemische davon. Geeignete Fettsäureester sind z.B. Methylpalmitat, Ethyloleat, Isopropylmyristat, n-Hexyllaurat, n-Butylstearat und Cetyl-/ Stearylisononanoat. Besonders bevorzugt sind Paraffinöle, Vaseline, Pflanzenöle, synthetische Triglyceride wie z.B. Glyceryltricaprylat, sowie Silikonöle.

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden. Als Konsistenzgeber kommen Fettalkohole mit 12 bis 22, vorzugsweise 12 bis 18 Kohlenstoffatomen sowie Partialglyceride in Betracht.

Als weitere Verdickungsmittel können Polysaccharide, insbesondere Xantham-Gum, Guar-Guar, Agar-Agar, Alginate, Carboxymethylcellulose, Hydroxyethylcellulose, höhermolekulare Polyethylenglykolmono- und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylpropan, Fettalkoholethoxylate oder Alkyloligoglucoside, sowie Elektrolyte wie Kochsalz und Ammoniumchlorid eingesetzt werden.

Geeignete Siliconverbindungen sind beispielsweise Dimethylpolysiloxan, Methylphenylpolysiloxane, cyclische Silicone sowie amino-, fettsäure-, alkohol-, polyether-, epoxy-, fluor- und/ oder alkylmodifizierte Siliconverbindungen, die bei Raumtemperatur sowohl flüssig als auch harzförmig vorliegen können.

Unter biogenen Wirkstoffen sind beispielsweise Bisabolol^{®}, Allantoin^{®}, Phytantriol^{®}, Panthenol^{®}, AHA-Säuren, Pflanzenextrakte und Vitaminkomplexe zu verstehen.

Als Antischuppenmittel können Climbazol^{®}, Octopirox^{®}, Oxiconazol^{®} und Zinkpyrethion^{®} eingesetzt werden.

Gebräuchliche Filmbildner sind Chitosan, mikrokristallines Chitosan, quatemiertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

Zur Verbesserung des Fließverhaltens können ferner Hydrotrope wie beispielsweise Ethanol, Isopropylalkohol, Propylenglykol oder Glucose eingesetzt werden.

Als Konservierungsmittel eignen sich beispielsweise Phenoxyethanol, Formaldehydlösung, Parabene, Pentandiol und Sorbinsäure.

Als feuchtigkeitsspendende Substanz stehen beispielsweise Isopropylpalmitat, Glycerin und/oder Sorbitol zu Verfügung.

Der Gesamtanteil an Hilfs- und Zusatzstoffen in den Mitteln beträgt bevorzugt 1 bis 50 Gew.-%, besonders bevorzugt 5 bis 40 Gew.-%.

Die nachfolgenden Beispiele dienen der Erläuterung der Erfindung, ohne sie jedoch einzuschränken. Der Quat-Aktivgehalt der erfindungsgemäßen
Zusammensetzungen wurde durch Kation-Titration bestimmt. Die Stockpunkte wurden durch langsames Absenken der Temperatur ermittelt.

### Beispiel 1:

110,54 g ^{®}Genamin KDMP (Behenyltrimethylammoniumchlorid Pellets mit einem Gehalt von ca. 19 % Isopropanol, Fa. Clariant GmbH) wurden in 38,37 g Dipropylenglykol in einem 1000 ml-Rundkolben gemischt und in einem Ölbad bei 70 - 90°C aufgeschmolzen. Nachdem sich eine homogene Schmelze gebildet hatte wurde am Rotationsverdampfer ein Vakuum von zunächst ca. 700 mbar angelegt und das Isopropanol während etwa 3 Stunden abdestilliert. Dabei wurde das Vakuum kontinuierlich auf etwa 22 mbar erhöht. Anschließend entfernte man Reste des flüchtigen Lösemittels während 2 Stunden bei 90°C Badtemperatur und 16 mbar Vakuum. Man erhielt eine klare Lösung, die bei 69 - 73°C erstarrte und bei 90°C wieder vollständig geschmolzen vorlag. Bei 25°C war die Zusammensetzung wachsartig fest. Der Quat-Aktivgehalt betrug 70 Gew.-%.

### Beispiel 2:

Analog Beispiel 1 wurde eine Zusammensetzung aus
75 Gew.-% Behenyltrimethylammoniumchlorid, 22 Gew.-% Dipropylenglykol und 3 Gew.-% Isopropanol hergestellt. Man erhielt eine klare Lösung, die bei ca. 73-77°C erstarrte und bei 90°C wieder vollständig geschmolzen vorlag. Bei 25°C war die Zusammensetzung wachsartig fest. Der Quat-Aktivgehalt betrug 75 Gew.-%.

### Beispiel 3:

Analog Beispiel 1 wurde eine Zusammensetzung aus
75 Gew.-% Behenyltrimethylammoniumchlorid, 23,7 Gew.-% 1,6-Hexandiol und 1,3 Gew.-% Isopropanol hergestellt. Man erhielt eine klare Lösung, die bei ca. 71 °C erstarrte und bei 90°C wieder vollständig geschmolzen vorlag. Bei 25°C war die Zusammensetzung wachsartig fest. Der Quat-Aktivgehalt betrug 75 Gew.-%.

### Beispiel 4:

^{®}Genamin KDMP (Behenyltrimethylammoniumchlorid Pellets mit einem Gehalt von ca. 19 % Isopropanol, Fa. Clariant GmbH) wurde in einem Vakuumofen zur Entfernung des Lösemittels 14 Stunden bei einer Temperatur 74°C und ca. 200 mbar Druck bis zur Gewichtskonstanz getrocknet (der Gewichtsverlust entsprach dabei der erwarteten Lösemittelmenge von ca. 19 %). Die so getrockneten Pellets wurden zu einem Pulver gemahlen und gesiebt (Siebweite: 630 µm).
4,9 g getrocknetes und gesiebtes Behenyltrimethylammoniumchlorid wurde in einer 50 ml-Pulverflasche aus Glas vorgelegt und mit 5,1 g Dipropylenglykol versetzt. Nach Verschließen der Flasche wurde die Mischung 4 Stunden auf ca. 100°C erhitzt. Während dieser Zeit wurde die Probe mit einem Spatel mehrmals gerührt, um die Mischung zu homogenisieren. Man erhielt eine klare Lösung, die bei ca. 45°C erstarrte und bei 70°C wieder vollständig geschmolzen vorlag. Der Quat-Aktivgehalt betrug 49 Gew.-%.

### Beispiel 5:

Analog Beispiel 4 wurde eine Zusammensetzung aus
49 Gew.-% Behenyltrimethylammoniumchlorid und 51 Gew.-% 1,6-Hexandiol hergestellt. Man erhielt eine klare Schmelze, die bei ca. 42°C erstarrte und bei 70°C wieder vollständig geschmolzen vorlag. Bei 25°C war die Zusammensetzung wachsartig fest. Der Quat-Aktivgehalt betrug 49 Gew.-%.

### Formulierungsbeispiele für kosmetische Formulierungen:

Die erfindungsgemäße Zusammensetzung aus Beispiel 3 wurde aufgeschmolzen und durch Auftropfen auf eine kalte Metallplatte pelletiert. Die so erhaltenen Pellets wurden dann in die entsprechenden Formulierungen eingearbeitet.

### Beispiel 6: Cremespülung

| | | |
|---|---|---|
| A | Zusammensetzung aus Bsp. 3 | 2 Gew.-% |
| | ^{®}HOSTAPHAT KL 340 D | 1,5 Gew.-% |
| | (Trilaureth-4 Phosphate, Fa. Clariant) Cetylalkohol Paraffinöl | 3 Gew.-% 1 Gew.-% |
| B | Wasser | 92,5 Gew.-% |
| C | Citronensäure | q.s. |

### Herstellung:

I) A bei 75°C aufschmelzen
II) B auf 75°C erwärmen
III) B in A einrühren und Abkühlen
IV) pH-Wert mit C auf pH = 4 einstellen

### Beispiel 7: O/W-Handcreme

| | | |
|---|---|---|
| A | Zusammensetzung aus Bsp. 3 | 2,7 Gew.-% |
| | ^{®}HOSTACERIN DGSB | 6 Gew.-% |
| | (PEG-4 Polyglyceryl-2 Stearate, Fa. Clariant) | |
| | Paraffinöl, hochviskos | 10 Gew.-% |
| | Isopropylpalmitat | 10 Gew.-% |
| | | |
| B | Wasser | 70,9 Gew.-% |
| | Konservierungsmittel | q.s. |
| C | Parfüm | 0,4 Gew.-% |

### Herstellung:

I) A bei 80°C aufschmelzen
II) B auf 80°C erwärmen
III) B in A einrühren und Abkühlen
IV) C bei 35°C zugeben

### Beispiel 8: Haarconditionierer mit Perlglanzeffekt

| | | |
|---|---|---|
| A | Zusammensetzung aus Bsp. 3 | 2 Gew.-% |
| | ^{®}Genamin KSL | 9 Gew.-% |
| | (PEG- 5 Stearyl Ammonium Lactate, Fa. Clariant) | |
| | ^{®}Hostaphat KL 340 D | 1,5 Gew.-% |
| | (Trilaureth- 4 Phosphate, Fa. Clariant) | |
| | Jojobaöl | 1,0 Gew.-% |
| | | |
| B | ^{®}Tylose H 100 000 YP2 | 1, 5 Gew.-% |
| | (Hydroxyethylcellulose, Fa. Clariant) | |
| C | Wasser | ad 100 % |
| | | |
| D | Parfüm | 0,50 % |
| | Panthenol | 0,50 % |
| | ^{®}Genapol PDC | 4 Gew.-% |
| | (Glycol Distearate, Laureth-4, Cocamidopropyl | |
| | Betaine, Glimmer und Titandioxid, Fa. Clariant) | |
| | | |
| E | Citronensäure | q.s. |

### Herstellung:

I) A wird auf 75°C erwärmt
II) B wird sorgfältig in C gelöst und auf etwa 75°C erwärmt
III) II wird unter Rühren zu I gegeben
IV) Unter Rühren abkühlen lassen, bei 30°C D in III geben
V) pH-Wert mit E auf pH=4 einstellen

## Patentansprüche

1. Zusammensetzungen, enthaltend
a) mindestens eine quaternäre Ammoniumverbindung gemäß Formel (1) 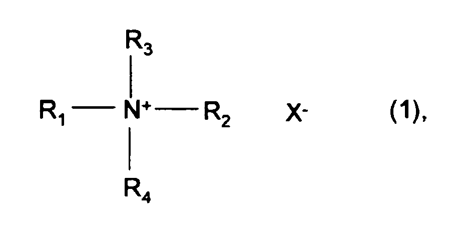 wobei
R₁ für eine unverzweigte oder verzweigte Alkyl- oder Alkenylgruppe mit 12 bis 36 Kohlenstoffatomen, eine Gruppe R₅CONH(CH₂)ₙ- oder eine Gruppe R₅COO(CH₂)ₙ-steht, wobei R₅ für eine Alkyl- oder Alkenylgruppe mit 12 bis 36 Kohlenstoffatomen und n für eine Zahl von 1 bis 8 stehen,
und
R₂, R₃ und R₄ unabhängig voneinander gleich oder verschieden sein können und für eine -CH₃, CH₃CH₂-, CH₃CH₂CH₂-, CH₃CH₂CH₂CH₂-, CH₂CH₂OH oder -CH₂CH(OH)CH₂OH-Gruppe stehen,
X⁻ für ein Anion steht,
und mindestens eine Ammoniumverbindung a) ausgewählt ist aus Alkyltrimethylammoniumverbindungen bei denen der Alkylrest einen Behenyl-, Erucyl-, Cetyl- oder Stearylrest darstellt,
und
b) mindestens einen mehrwertigen Alkohol mit 5 bis 12 Kohlenstoffatomen,
**dadurch gekennzeichnet, dass** der Gehalt an quaternären Ammoniumverbindungen a), bezogen auf die fertigen Zusammensetzungen, 60 bis 90 Gew.-% beträgt und die Zusammensetzungen in Form von Pellets oder Flakes vorliegen, mit der Maßgabe, dass die Zusammensetzungen frei sind von verzweigten Alkoholen b1) mit 8 bis 36 Kohlenstoffatomen oder Mischungen aus mindestens einem verzweigten Alkohol b1) und mindestens einem unverzweigten Alkohol b2) mit 8 bis 36 Kohlenstoffatomen, wenn sie mindestens einen mehrwertigen Alkohol mit 2 bis 6 Kohlenstoffatomen enthalten.

2. Zusammensetzungen nach Anspruch 1, **dadurch gekennzeichnet, dass** sie frei sind von Fettalkoholen und unverzweigten oder verzweigten Monoalkoholen mit 8 bis 36 Kohlenstoffatomen.

3. Zusammensetzungen nach Anspruch 1 und/oder 2, **dadurch gekennzeichnet, dass** der Gehalt an quaternären Ammoniumverbindungen a), bezogen auf die fertigen Zusammensetzungen, 60 bis 85 Gew.-%, beträgt.

4. Zusammensetzungen nach mindestens einem der Ansprüche 1 bis 3,
**dadurch gekennzeichnet, dass** es sich bei den quaternären Ammoniumverbindungen a) um (C₁₂-C₃₆)-, bevorzugt (C₁₄-C₃₀)-, besonders bevorzugt (C₁₆-C₂₄)-Alkyltrimethylammoniumverbindungen, handelt.

5. Zusammensetzungen nach mindestens einem der Ansprüche 1 bis 4,
**dadurch gekennzeichnet, dass** es sich beim Anion X⁻ in Formel (1) um Chlorid, lodid, Bromid, Methosulfat, Hydrogensulfat, Lactat und/oder Citrat, bevorzugt Chlorid und/oder Methosulfat, handelt.

6. Zusammensetzungen nach mindestens einem der Ansprüche 1 bis 5,
**dadurch gekennzeichnet, dass** mindestens eine Ammoniumverbindung a) ausgewählt ist aus Alkyltrimethylammoniumverbindungen bei denen der Alkylrest einen Behenyl-, Erucyl- oder Stearylrest darstellt.

7. Zusammensetzungen nach mindestens einem der Ansprüche 1 bis 6,
**dadurch gekennzeichnet, dass** der Gehalt an mehrwertigen Alkoholen b), bezogen auf die fertigen Zusammensetzungen, 10 bis 70 Gew.-%, bevorzugt 15 bis 60 Gew.-%, besonders bevorzugt 15 bis 55 Gew.-%, beträgt.

8. Zusammensetzungen nach mindestens einem der Ansprüche 1 bis 7,
**dadurch gekennzeichnet, dass** es sich bei den mehrwertigen Alkoholen b) um Pentandiol, Hexandiol, Hexylenglykol, Trimethylpentandiol, Heptandiol, Octandiol, Nonandiol, Decandiol, Undecandiol, Dodecandiol, Diglycerin, Triglycerin, Dipropylenglykol, Tripropylenglykol, Sorbitol, Xylitol, Mannitol und/oder Mischungen derselben handelt.

9. Zusammensetzungen nach Anspruch 8, **dadurch gekennzeichnet, dass** es sich bei den mehrwertigen Alkoholen b) um 1,5-Pentandiol, 1,2-Pentandiol, 1,6-Hexandiol, 1,2-Hexandiol, 1,7-Heptandiol, 1,2-Heptandiol, 1,8-Octandiol, 1,2-Octandiol, 1,9-Nonandiol, 1,2-Nonandiol, 1,10-Decandiol, 1,2-Decandiol, 1,11-Undecandiol, 1,2-Undecandiol, 1,12-Dodecandiol, 1,2-Dodecandiol, 2-Methyl-2,4-pentandiol, 2,2,4-Trimethyl-1,3-pentandiol, Diglycerin, Triglycerin, Dipropylenglykol, Tripropylenglykol, Sorbitol, Xylitol, Mannitol und/oder Mischungen derselben handelt.

10. Zusammensetzungen nach Anspruch 9, **dadurch gekennzeichnet, dass** es sich bei den mehrwertigen Alkohole b) um 1,6-Hexandiol, 2-Methyl-2,4-pentandiol, 2,2,4-Trimethyl-1,3-pentandiol und/oder Dipropylenglykol handelt.

11. Zusammensetzungen nach mindestens einem der Ansprüche 1 bis 9,
**dadurch gekennzeichnet, dass** sie mindestens einen mehrwertigen Alkohol b) mit 6 bis 8 Kohlenstoffatomen enthalten.

12. Zusammensetzungen nach mindestens einem der Ansprüche 1 bis 11,
**dadurch gekennzeichnet, dass** sie, bezogen auf die fertigen Zusammensetzungen, weniger als 5 Gew.-%, bevorzugt weniger als 3 Gew.-%, an unverzweigten oder verzweigten Monoalkoholen mit 1 bis 4 Kohlenstoffatomen enthalten.

13. Zusammensetzungen nach Anspruch 12, **dadurch gekennzeichnet, dass** es sich bei den Monoalkoholen um Ethanol, Propanol, Isopropanol, Butanol, Isobutanol und t-Butanol, bevorzugt Isopropanol, handelt.

14. Zusammensetzungen nach mindestens einem der Ansprüchen 1 bis 13, **dadurch gekennzeichnet, dass** sie Stockpunkte unterhalb 100°C, bevorzugt unterhalb 95°C, besonders bevorzugt unterhalb 90°C, insbesondere bevorzugt unterhalb 85°C, besitzen.

15. Zusammensetzungen nach mindestens einem der Ansprüche 1 bis 14,
**dadurch gekennzeichnet, dass** sie Flammpunkte oberhalb 80°C, bevorzugt oberhalb 100°C, besitzen.

16. Zusammensetzungen nach mindestens einem der Ansprüche 1 bis 15,
**dadurch gekennzeichnet, dass** die Zusammensetzungen in Form von Pellets vorliegen.

17. Verfahren zur Herstellung von Zusammensetzungen gemäß mindestens einem der Ansprüche 1 bis 16, **dadurch gekennzeichnet, dass** man mindestens eine quaternäre Ammoniumverbindung a), gegebenenfalls enthaltend einen verzweigten oder unverzweigten Monoalkohol mit 1 bis 4 Kohlenstoffatomen, und mindestens einen mehrwertigen Alkohol mit 5 bis 12 Kohlenstoffatomen und gegebenenfalls mindestens einen unverzweigten oder verzweigten Monoalkohol mit 1 bis 4 Kohlenstoffatomen mischt und diese Mischung in Form von Pellets oder Flakes bringt.

18. Verfahren zur Herstellung einer Zusammensetzung gemäß mindestens einem der Ansprüche 1 bis 16, **dadurch gekennzeichnet, dass** man
i) mindestens ein tertiäres Amin NR₁R₂R₃, wobei
R₁ für eine unverzweigte oder verzweigte Alkyl- oder Alkenylgruppe mit 12 bis 36 Kohlenstoffatomen, eine Gruppe R₅CONH(CH₂)ₙ- oder eine Gruppe R₅COO(CH₂)ₙ- steht, wobei R₅ für eine Alkyl- oder Alkenylgruppe mit 12 bis 36 Kohlenstoffatomen und n für eine Zahl von 1 bis 8 stehen,
und R₂ und R₃ unabhängig voneinander gleich oder verschieden sein können und für -CH₃, CH₃CH₂-, CH₃CH₂CH₂-, CH₃CH₂CH₂CH₂- oder -CH₂CH₂(OH) stehen,
durch
ii) mindestens ein Alkylierungsmittel, ausgewählt aus
a) R₄X, wobei R₄ für -CH₃-, CH₃CH₂-, CH₃CH₂CH₂-, CH₃CH₂CH₂CH₂- oder -CH₂CH(OH)CH₂(OH) steht und X für Cl, 1, Br, OSO₃H oder Methosulfat steht, und/oder
b) Ethylenoxid und einer Säure HX, wobei X für CI, I, Br, OSO₃H, Citrat oder Lactat steht,
in Gegenwart von
iii) mindestens einem mehrwertigen Alkohol mit 5 bis 12 Kohlenstoffatomen und
iv) gegebenenfalls mindestens einem unverzweigten oder verzweigten Monoalkohol mit 1 bis 4 Kohlenstoffatomen
alkyliert und anschließend in Form von Pellets oder Flakes bringt.

19. Verwendung von Zusammensetzungen gemäß mindestens einem der Ansprüche 1 bis 16 zur Herstellung von kosmetischen, dermatologischen und pharmazeutischen Mitteln, bevorzugt Haarbehandlungsmitteln.

20. Verwendung nach Anspruch 19, **dadurch gekennzeichnet, dass** es sich bei den Haarbehandlungsmitteln um Shampoos, rinse-off-Haarconditioner, Cremespülungen, Klarspülungen, Haarkuren, Haarfärbe- und Haartönungsmittel, Dauerwellmittel, Haargele oder Haarconditioner in Aerosol-, Spray- und Fluidform handelt.

## Claims

1. A composition comprising
a) at least one quaternary ammonium compound according to formula (1) 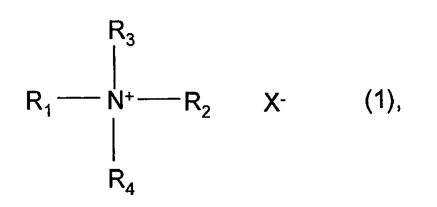 where
R₁ is an unbranched or branched alkyl or alkenyl group having 12 to 36 carbon atoms, a group R₅CONH (CH₂) ₙ- or a group R₅COO(CH₂)ₙ-, where R₅ is an alkyl or alkenyl group having 12 to 36 carbon atoms and n is a number from 1 to 8,
and
R₂, R₃ and R₄, independently of one another, may be identical or different and are a -CH₃, CH₃CH₂-, CH₃CH₂CH₂-, CH₃CH₂CH₂CH₂-, CH₂CH₂OH or -CH₂CH (OH) CH₂OH group,
X⁻ is an anion,
and at least one ammonium compound a) is seleced from alkyltrimethylammonium compounds in which the alkyl radical is a behenyl, erucyl, cetyl or stearyl radical,
and
b) at least one polyhydric alcohol having 5 to 12 carbon atoms,
wherein the content of quaternary ammonium compounds a), based on the finished composition, is 60 to 90% by weight and the composition is in the form of pellets or flakes, with the proviso that the composition is free of branched alcohols b1) having 8 to 36 carbon atoms or mixtures of at least one branched alcohol b1) and at least one unbranched alcohol b2) having 8 to 36 carbon atoms, when it contains at least one polyhydric alcohol having 2 to 6 carbon atoms.

2. The composition as claimed in claim 1, which is free from fatty alcohols and unbranched or branched monoalcohols having 8 to 36 carbon atoms.

3. The composition as claimed in claim 1 and/or 2,
wherein the content of quaternary ammonium compounds a), based on the finished composition, is 60 to 85% by weight.

4. The composition as claimed in at least one of claims 1 to 3, wherein the quaternary ammonium compounds a) are (C₁₂-C₃₆) -, preferably (C₁₄-C₃₀)-. particularly preferably (C₁₆-C₂₄) -alkyltrimethylammonium compounds.

5. The composition as claimed in at least one of claims 1 to 4, wherein the anion X⁻ in formula (1) is chloride, iodide, bromide, methosulfate, hydrogensulfate, lactate and/or citrate, preferably chloride and/or methosulfate.

6. The composition as claimed in at least one of claims 1 to 5, wherein at least one ammonium compound a) is selected from alkyltrimethylammonium compounds in which the alkyl radical is a behenyl, erucyl or stearyl radical.

7. The composition as claimed in at least one of claims 1 to 6, wherein the content of polyhydric alcohols b), based on the finished composition, is 10 to 70% by weight, preferably 15 to 60% by weight, particularly preferably 15 to 55% by weight.

8. The composition as claimed in at least one of claims 1 to 7, wherein the polyhydric alcohols b) are pentanediol, hexanediol, hexylene glycol, trimethylpentanediol, heptanediol, octanediol, nonanediol, decanediol, undecanediol, dodecanediol, diglycerol, triglycerol, dipropylene glycol, tripropylene glycol, sorbitol, xylitol, mannitol and/or mixtures thereof.

9. The composition as claimed in claim 8, wherein the polyhydric alcohols b) are 1,5-pentanediol, 1,2-pentanediol, 1,6-hexanediol, 1,2-hexanediol, 1,7-heptanediol, 1,2-heptanediol, 1,8-octanediol, 1,2-octanediol, 1,9-nonanediol, 1,2-nonanediol, 1,10-decanediol, 1,2-decanediol, 1,11-undecanediol, 1,2-undecanediol, 1,12-dodecanediol, 1,2-dodecanediol, 2-methyl-2,4-pentanediol, 2,2,4-trimethyl-1,3-pentanediol, diglycerol, triglycerol, dipropylene glycol, tripropylene glycol, sorbitol, xylitol, mannitol and/or mixtures thereof.

10. The composition as claimed in claim 9, wherein the polyhydric alcohols b) are 1,6-hexanediol, 2-methyl-2,4-pentanediol, 2,2,4-trimethyl-1,3-pentanediol and/or dipropylene glycol.

11. The composition as claimed in at least one of claims 1 to 9, which comprises at least one polyhydric alcohol b) having 6 to 8 carbon atoms.

12. The composition as claimed in at least one of claims 1 to 11, which comprises, based on the finished composition, less than 5% by weight, preferably less than 3% by weight, of unbranched or branched monoalcohols having 1 to 4 carbon atoms.

13. The composition as claimed in claim 12, wherein the monoalcohols are ethanol, propanol, isopropanol, butanol, isobutanol and tert-butanol, preferably isopropanol.

14. The composition as claimed in at least one of claims 1 to 13, which has a setting point below 100°C, preferably below 95°C, particularly preferably below 90°C, in particular below 85°C.

15. The composition as claimed in at least one of claims 1 to 14, which has a flash point above 80°C, preferably above 100°C.

16. The composition as claimed in at least one of claims 1 to 15, which is in the form of pellets.

17. A process for the preparation of compositions as claimed in at least one of claims 1 to 16, which comprises mixing at least one quaternary ammonium compound a), optionally containing a branched or unbranched monoalcohol having 1 to 4 carbon atoms, and at least one polyhydric alcohol having 5 to 12 carbon atoms and optionally at least one unbranched or branched monoalcohol having 1 to 4 carbon atoms and bringing said mixture into the form of pellets or flakes.

18. A process for the preparation of a composition as claimed in at least one of claims 1 to 16, which comprises alkylating
i) at least one tertiary amine NR₁R₂R₃, where
R₁ is an unbranched or branched alkyl or alkenyl group having 12 to 36 carbon atoms, a group R₅CONH (CH₂)ₙ- or a group R₅COO(CH₂)ₙ-, where R₅ is an alkyl or alkenyl group having 12 to 36 carbon atoms and n is a number from 1 to 8,
and R₂ and R₃, independently of one another, may be identical or different and are -CH₃, CH₃CH₂-, CH₃CH₂CH₂-, CH₃CH₂CH₂CH₂- or -CH₂CH₂(OH),
by
ii) at least one alkylating agent chosen from
a) R₄X, where R₄ is -CH₃-, CH₃CH₂-, CH₃CH₂CH₂-, CH₃CH₂CH₂CH₂- or -CH₂CH(OH)CH₂(OH), and X is Cl, I, Br, OSO₃H or methosulfate, and/or
b) ethylene oxide and an acid HX, where X is Cl, I, Br, OSO₃H, citrate or lactate,
in the presence of
iii) at least one polyhydric alcohol having 5 to 12 carbon atoms and
iv) optionally at least one unbranched or branched monoalcohol having 1 to 4 carbon atoms
and subsequently bringing the alkylation product into the form of pellets or flakes.

19. The use of compositions as claimed in at least one of claims 1 to 16 for the preparation of cosmetic, dermatological and pharmaceutical compositions, preferably hair-treatment compositions.

20. The use as claimed in claim 19, wherein the hair-treatment compositions are shampoos, rinse-off hair conditioners, cream rinses, clear rinses, hair cures, hair colorants and hair tints, permanent waving compositions, hair gels and hair conditioners in aerosol form, spray form and fluid form.

## Revendications

1. Compositions, contenant
a)au moins un composé d'ammonium quaternaire selon la formule (1) 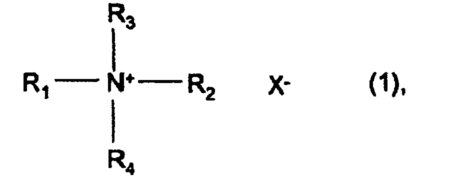 dans laquelle
R₁ représente un groupe alkyle ou alcényle ramifié ou non ramifié ayant de 12 à 36 atomes de carbone, un groupe R₅CONH (CH₂)ₙ- ou un groupe R₅COO (CH₂)ₙ-, R₅ représentant un groupe alkyle ou alcényle ayant de 12 à 36 atomes de carbone et n représentant un nombre valant de 1 à 8,
et
R₂, R₃ et R₄ chacun indépendamment peuvent être identiques ou différents et représentent un groupe -CH₃, CH₃CH₂-, CH₃CH₂CH₂-, CH₃CH₂CH₂CH₂-, CH₂CH₂OH ou -CH₂CH(OH)CH₂OH,
X⁻ représente un anion,
et au moins un composé d'ammonium a) est choisi parmi les composés alkyltriméthylammonium dans lesquels le radical alkyle représente un radical béhényle, érucyle, cétyle ou stéaryle,
et
b) au moins un alcool polyhydrique ayant de 5 à 12 atomes de carbone, **caractérisées en ce que** la teneur en composés d'ammonium quaternaire a), par rapport aux compositions finales, va de 60 à 90 % en poids et les compositions se trouvent sous forme de granules ou de paillettes, étant entendu que les compositions sont exemptes d'alcools b1) ramifiés ayant de 8 à 36 atomes de carbone ou de mélanges d'au moins un alcool b1) ramifié et d'au moins un alcool b2) non ramifié ayant de 8 à 36 atomes de carbone lorsqu'elles contiennent au moins un alcool polyhydrique ayant de 2 à 6 atomes de carbone.

2. Compositions selon la revendication 1,
**caractérisées en ce qu'**elles sont exemptes d'alcools gras et de monoalcools ramifiés ou non ramifiés ayant de 8 à 36 atomes de carbone.

3. Compositions selon la revendication 1 et/ou la revendication 2, **caractérisées en ce que** la teneur en composés d'ammonium quaternaire a), par rapport aux compositions finales, va de 60 à 85 % en poids.

4. Compositions selon au moins l'une des revendications 1 à 3, **caractérisées en ce que** les composés d'ammonium quaternaire a) consistent en des composés alkyl (C₁₂-C₃₆)-, de préférence (C₁₄-C₃₀) -, de façon particulièrement préférée (C₁₆-C₂₄)-triméthylammonium.

5. Compositions selon au moins l'une des revendications 1 à 4, **caractérisées en ce que** l'anion X⁻ dans la formule (1) consiste en chlorure, iodure, bromure, méthosulfate, hydrogénosulfate, lactate et/ou citrate, de préférence en chlorure et/ou méthosulfate.

6. Compositions selon au moins l'une des revendications 1 à 5, **caractérisées en ce qu'**au moins un composé ammonium a) est choisi parmi les composés alkyltriméthylammonium dans lesquels le radical alkyle représente un radical béhényle, érucyle ou stéaryle.

7. Compositions selon au moins l'une des revendications 1 à 6, **caractérisées en ce que** la teneur en alcools polyhydriques b), par rapport aux compositions finales, va de 10 à 70 % en poids, de préférence de 15 à 60 % en poids, de façon particulièrement préférée de 15 à 55 % en poids.

8. Compositions selon au moins l'une des revendications 1 à 7, **caractérisées en ce que** les alcools polyhydriques b) consistent en pentanediol, hexanediol, hexylèneglycol, triméthylpentanediol, heptanediol, octanediol, nonanediol, décanediol, undécanediol, dodécanediol, diglycérol, triglycérol, dipropylèneglycol, tripropylèneglycol, sorbitol, xylitol, mannitol et/ou en des mélanges de ceux-ci.

9. Compositions selon la revendication 8,
**caractérisées en ce que** les alcools polyhydriques b) consistent en 1,5-pentanediol, 1,2-pentanediol, 1,6-hexanediol, 1,2-hexanediol, 1,7-heptanediol, 1,2-heptanediol, 1,8-octanediol, 1,2-octanediol, 1,9-nonanediol, 1,2-nonanediol, 1,10-décanediol, 1,2-décanediol, 1,11-undécanediol, 1,2-undécanediol, 1,12-dodécanediol, 1,2-dodécanediol, 2-méthyl-2,4-pentandediol, 2,2,4-triméthyl-1,3-pentanediol, diglycérol, triglycérol, dipropylèneglycol, tripropylèneglycol, sorbitol, xylitol, mannitol et/ou en des mélanges de ceux-ci.

10. Compositions selon la revendication 9,
**caractérisées en ce que** les alcools polyhydriques b) consistent en 1,6-hexanediol, 2-méthyl-2,4-pentanediol, 2,2,4-triméthyl-1,3-pentanediol et/ou dipropylèneglycol.

11. Compositions selon au moins l'une des revendications 1 à 9, **caractérisées en ce qu'**elles contiennent au moins un alcool polyhydrique b) ayant de 6 à 8 atomes de carbone.

12. Compositions selon au moins l'une des revendications 1 à 11, **caractérisées en ce qu'**elles contiennent, par rapport aux compositions finales, moins de 5 % en poids, de préférence moins de 3 % en poids, de monoalcools ramifiés ou non ramifiés ayant de 1 à 4 atomes de carbone.

13. Compositions selon la revendication 12,
**caractérisées en ce que** les monoalcools consistent en éthanol, propanol, isopropanol, butanol, isobutanol et tert-butanol, de préférence en isopropanol.

14. Compositions selon au moins l'une des revendications 1 à 13, **caractérisées en ce qu'**elles présentent des points de solidification inférieurs à 100 °C, de préférence inférieurs à 95 °C, de façon particulièrement préférée inférieurs à 90 °C, de façon tout particulièrement préférée inférieurs à 85 °C.

15. Compositions selon au moins l'une des revendications 1 à 14, **caractérisées en ce qu'**elles présentent des points d'inflammation supérieurs à 80 °C, de préférence supérieurs à 100 °C.

16. Compositions selon au moins l'une des revendications 1 à 15, **caractérisées en ce que** les compositions se trouvent sous forme de granules.

17. Procédé pour la préparation de compositions selon au moins l'une des revendications 1 à 16,
**caractérisées en ce qu'**on mélange au moins un composé d'ammonium quaternaire a), contenant éventuellement un monoalcool ramifié ou non ramifié ayant de 1 à 4 atomes de carbone, et au moins un alcool polyhydrique ayant de 5 à 12 atomes de carbone et éventuellement au moins un monoalcool ramifié ou non ramifié ayant de 1 à 4 atomes de carbone et on met ce mélange sous forme de granules ou de paillettes.

18. Procédé pour la préparation d'une composition selon au moins l'une des revendications 1 à 16,
**caractérisé en ce qu'**on soumet à une alkylation
i) au moins une amine tertiaire NR₁R₂R₃, dans laquelle
R₁ représente un groupe alkyle ou alcényle ramifié ou non ramifié ayant de 12 à 36 atomes de carbone, un groupe R₅CONH (CH₂) ₙ- ou un groupe R₅COO (CH₂)ₙ-,
R₅ représentant un groupe alkyle ou alcényle ayant de 12 à 36 atomes de carbone et n représentant un nombre valant de 1 à 8,
et R₂ et R₃, indépendamment l'un de l'autre, peuvent être identiques ou différents et représentent un groupe -CH₃, CH₃CH₂-, CH₃CH₂CH₂-, CH₃CH₂CH₂CH₂- ou -CH₂CH₂(OH),
par
ii) au moins un agent d'alkylation choisi parmi
a) R₄X, R₄ représentant un groupe -CH₃, CH₃CH₂-, CH₃CH₂CH₂-, CH₃CH₂CH₂CH₂- ou -CH₂CH (OH) CH₂ (OH) et X représentant Cl, I, Br, OSO₃H ou l'ion méthosulfate, et/ou
b) l'oxyde d'éthylène et un acide HX, représentant Cl, I, Br, OSO₃H, l'ion citrate ou lactate,
en présence
iii) d'au moins un alcool polyhydrique ayant de 5 à 12 atomes de carbone et
iv) éventuellement d'au moins un monoalcool ramifié ou non ramifié ayant de 1 à 4 atomes de carbone
et on la met ensuite sous forme de granules ou de paillettes.

19. Utilisation de compositions selon au moins l'une des revendications 1 à 16, pour la fabrication de produits cosmétiques, dermatologiques et pharmaceutiques, de préférence de produits de traitement capillaire.

20. Utilisation selon la revendication 19, **caractérisée en ce que** les produits de traitement capillaire sont des shampooings, des conditionneurs pour cheveux à éliminer par rinçage (*rinse-off*), des produits de rinçage sous forme de crème, des produits de rinçage liquides, des lotions de traitement capillaire, des produits de teinture et de nuançage pour cheveux, des produits pour ondulation permanente, des gels capillaires ou des conditionneurs pour cheveux sous forme d'aérosols, de laques ou de liquides.
